# EUROPEAN PATENT SPECIFICATION

(11) **EP 2 999 441 B1**
(45) Date of publication and mention of the grant of the patent: **20.09.2017**
(21) Application number: 14739507.3
(22) Date of filing: 23.05.2014
(51) Int. Cl.: A61F 7/02, A61H 9/00, A61F 7/00

(54) **COMPRESSION AND THERMOREGULATION DEVICE**
KOMPRESSIONS- UND WÄRMEREGELUNGSVORRICHTUNG
DISPOSITIF DE COMPRESSION ET DE THERMORÉGULATION

(30) Priority: 23.05.2013 GB 201309310
(43) Date of publication of application: 30.03.2016
(73) Proprietor: Physiolab Technologies Limited, Colchester Essex C03 3BZ (GB)
(72) Inventor: ROSE, Nicholas, Colchester Essex CO3 3BZ (GB)
(74) Representative: Williams Powell
(86) International application number: PCT/GB2014/051595
(87) International publication number: WO 2014/188213

(56) References cited:
- EP-A2- 1 972 312
- WO-A1-93/09737
- US-A- 3 871 381
- US-A1- 2007 135 720
- US-A1- 2009 312 676
- US-A1- 2012 022 416
- US-B1- 6 551 280

## Description

### Technical Field

The present invention relates to a device for heating or cooling a body part for the treatment of physical injuries and disorders. Optionally, the device can also provide compression to a body part.

### Background Art

It has been known for a number of years that physical injuries (for example injuries sustained during sport) can be treated by applying compression to the injury and cooling it below ambient temperatures (so-called cold pressure therapy). In simple terms, the thermal cooling tends to reduce the inflammatory response and the pain from the heat generated by the injury and the compression seems to reduce the swelling caused by fluid acumination beneath the skin.

Thermal treatments of this type have been known for many years, in their simplest form being ice packs. Subsequently, heat generating packs were developed, typically in the form of a pouch of chemical material which can be made to react exothermically and thereby to release heat. These devices are intended to cool or heat, as appropriate, a part of a person's body in order to alleviate inflammation, pain suffered by the patient and so on. It has been found that if properly applied, such treatments can significantly minimise patient discomfort caused by the injury or illness as well as speed up the recovery process. However, such ice packs and heat generating packs provide a relatively crude form of temperature regulation, unable to provide optimum treatment of an injury without constant and close monitoring by a medical practitioner.

Traditional methods of providing compression have been equally crude. In its simplest from, compression can be applied simply by the patience or the medic pushing the ice pack (for example) into the body part to be treated. A slightly more sophisticated method may involve strapping a thermal regulation pack onto the patient's limb by means of Velcro® attachments, for example. However, these attachment methods have a number of significant disadvantages when it comes to treatment.

First, it has been found that the act of wrapping a flat treatment pack around a three-dimensional limb results in an uneven heating/cooling effect. This is particularly true when the heating or cooling is provided by the flow of a heating or cooling fluid along channels disposed in the treatment pack, because the act of wrapping the pack around the limb and securing it causes uneven compression of the pack and uneven impediment of fluid flow within the channels. Thus, at worse, a situation may arise in which the limb or a part of the limb is not being cooled/heated at all, or at the very least the limb is not being heated/cooled in an even and controllable manner, thereby reducing the effectiveness of the treatment.

Secondly, the wrapping of a flat treatment pack around a three-dimensional limb does not enable an even and controllable compression to be applied to the limb. It will be appreciated that the compression which is applied is a function of how tightly the pack is wrapped around the limb, and this depends upon who has applied the pack and how tightly they have wrapped it. In most instances, the compression is not repeatable even if the same person in applying the pack in each case, and certainly not if different people are applying the pack. Furthermore, the compression may vary at different points along the limb depending on how evenly the pack has been wrapped.

Some treatment packs apply pressure to the patient by means of a compression fluid (such as air) which is provided under pressure into a chamber in the pack such that the pressure in the air in the chamber pushes the cooling or heating part of the pack into the patient's limb. However, these suffer from the same disadvantage as mentioned above, in that the wrapping of a flat pack around a three-dimensional limb results in a situation which the flow of pressurised air around the pack cannot be properly controlled or predicted and thus the compression effect is undesirably variable.

Finally, compression packs which rely upon the tightness of the wrapping to produce the compression are limited in application in that the compression cannot be varied over time as part of the treatment regime.

There have been a number of attempts to design cuffs suited to such thermoregulation systems, including for instance EP-1,929,980 and US 2006/0191675. However, these designs do not generally resolve the problems indicated above.

US 6,656,208 (Grahn et al) discloses a sleeve with one closed end into which the arm of a user can be placed and cooled under negative pressure conditions.

US 6,846,322 (Kane et al) discloses a rigid clam-shell unit into which a user's arm can be placed for cooling whilst a vacuum is applied.

US 2008/0132976 (Kane) discloses an enclosed space in the form of a "glove" for a patient's hand and arm. The device has a thermal exchange unit and an internal region which can be evacuated to enclose and compress the thermal exchange unit against the arm.

US 2008/0021531 (Kane) discloses apparatus for the prevention of deep vein thrombosis and associated medical conditions in which the temperature of the body part is adjusted and/or a vacuum is applied to increase blood flow.

JP 8/131473 (Daikin Ind Ltd) discloses a head cooling device with a cooling part and a pressurising part which presses the cooling part into the head and neck of the user.

US 2009/312676 A1 (Rousso et al.) discloses a method for increasing metabolism of a subject's body in order to lose weight, comprising: a) contacting a part of the body with a cooling element to remove heat from the body; and b) repeating or continuing (a) so as to remove enough heat in total to lose at least 1 kilogram of body weight. Disclosed devices for carrying out the method include cylindrical cooling devices into which a body part can be placed.

EP 1 972 312 A2 (Dynatherm Medical Inc.) discloses apparatus for adjusting blood circulation including one or more collapsible and pliant body element capable of expanding and flexibly applying pressurised compression forces to user body parts.

WO 93/09737 (P.I. Inc.) discloses a thermal pack for heating or cooling portions of the body which includes a gel pad which can be heated or cooled and a pressure chamber.

### Summary of the Present Invention

In accordance with a first aspect of the invention, there is provided a device for heating or cooling a body part, comprising a body having an elongate treatment cavity into which a body part can be placed for treatment, a first chamber having an inlet and an outlet for a heating or cooling fluid, wherein the first chamber is formed by a space between an inner wall of the first chamber in the form of a cylindrical surface and an outer wall of the first chamber in the form of a cylindrical surface, wherein the inner wall of the first chamber forms the external boundary of at least part of the treatment cavity, whereby heating or cooling fluid in the first chamber heats or cools a body part in the treatment cavity, and wherein the treatment cavity is open at both ends so that in use a body part can be placed into one end of the treatment cavity and emerge from the other end characterised in that the device additionally comprises means for compressing, in use, the first chamber into the body part, wherein the means for compressing comprises a second chamber completely surrounding the first chamber and having an inlet and an outlet for a pressurising fluid, wherein the second chamber is formed by a space between an inner wall of the second chamber in the form of a cylindrical surface and an outer wall of the second chamber in the form of a cylindrical surface, whereby putting said fluid under pressure in the second chamber compresses the first chamber into the body part.

The fact that the second chamber(s) continuously surrounds the first chamber means that in use the pressure exerted by the second chamber against the first chamber and thereby against the body part is even and controllable in nature. In contrast to known "wrap-around" devices, it avoids the need for the user or medical advisor to have to judge how tightly to exert pressure, and because there is no need to wrap a two-dimensional chamber around a three-dimensional body part, there are no boundaries to disrupt the flow of pressurised air around the chamber, because the air can flow in an even and unimpeded fashion.

When the second chamber is pressurised to compress the first chamber around the body part, there may be excess material resulting from the outer wall of the first chamber having a greater circumference than the circumference of the body part. A further technical advantage of the device is that this excess material can be accommodated in the second chamber in the form of ridges, wrinkles or rucks, without this excess material impeding the flow of fluid within the first chamber. This "ruckling" effect has the dual advantage of dynamically conforming the device to the body part and maximising the surface area of the cooling or heating effect.

The technical advantages of the treatment cavity being open at both ends are first that the user can choose the position at which the therapy is applied on the limb more easily, and secondly that all compression forces (if applied) are directed inwardly into the limb rather than some being directed axially as in "closed end" devices.

The means for compressing may also comprise
a thicker or relatively inelastic outer wall for the first chamber, which forces the first chamber into the body part when the first chamber is pressurised.
There may be radial partitions to form a plurality of second chambers aligned longitudinally along the device, each of which encircles the first chamber.

In a preferred embodiment, the first chamber continuously surrounds the space into which the body part can be situated in use. This means that, in use, the heating or cooling effect of the first fluid on the body part is even, without there being any discontinuities caused by boundaries within the first chamber which impede the flow of the first fluid. In essence, the device allows for a total submersion of the body part in the heating or cooling fluid, whilst also providing an even and controllable compression of the body part.

The first chamber is formed by the space between an inner wall in the form of a cylindrical surface (such as a tube) and an outer wall in the form of a cylindrical surface (such as a tube). Preferably, the physical properties of said inner wall are the same as the physical properties of said outer wall. Most preferable, the cylinders bounded by said inner wall and said outer wall are concentric.

The space into which the body part can be situated is preferably a cylindrical space bounded by the first chamber's inner wall. This cylindrical space is ideally suited to house a body part such as an arm or leg.

The second chamber is formed by the space between an inner wall in the form of a cylindrical surface (such as a tube) and an outer wall in the form of a cylindrical surface (such as a tube). The outer wall is preferably thicker and/or less elastic than the inner wall, such that the force of the pressurised second fluid is directed inwardly towards the first chamber and the body part. Again, the cylinders bounded by said inner wall and said outer wall are preferably concentric.

In a preferred embodiment, the inner wall of the second chamber is formed from the outer wall of the first chamber.

The first chamber preferably includes a material through which the first fluid can flow in use. More preferably, this material has multiple pathways (which may be equally deformable) therethrough for the first fluid which enable even distribution of the fluid through the chamber. Examples of such a material include a plurality of particles (such as polyballs), a 3D fabric (spacer fabric), a fluid (such as a gas or a viscous liquid) or (most preferably) an open-celled foam.

In a preferred embodiment, an open-celled foam having a porosity of from 5 to 15, more preferably about 10 pores per inch (PPI) is employed, such as a 6mm thick reticulated polyurethane foam. The advantage of this porosity is that that the cuff does not take on too much liquid and instead creates thin randomised channels close to the tissue.

The provision of such a material has the advantages of taking up space in the first chamber (so that a smaller volume of the first fluid is required), maintaining the shape and structure of the chamber, and ensuring that fluid flow is evenly distributed. This improves the even cooling or heating effect of the device.

The device may additionally comprise a first passage along which fluid can flow to the first end of the first chamber, and a second passage along which fluid can flow from the second end of the first chamber. The first passage preferably connects the inlet and the first end of the first chamber and the second passage preferably connects the second end and the outlet of the second chamber.

The device may also include a sleeve into which the second and first chambers can be housed in use, which sleeve is formed of at least two sections having different physical properties (such as flexibility, expandability and diameter of the sleeve), whereby in use the section with the most appropriate physical property can be aligned with a specific part of the body to be treated.

Preferably, the sleeve has two end sections and a middle section and wherein the middle section is more flexible than the two end sections whereby in use the middle section can be aligned with a joint of a limb so that the sleeve can be flexed at the middle section when the joint is flexed.

### Brief Description of the drawings

A number of preferred embodiments of the invention will now be described, with reference to the accompanying drawings, in which:
Figure 1 is a schematic diagram of a patient wearing a number of devices according to the invention;
Figure 2 is a cross-sectional schematic diagram of a device according to the invention worn on a body part;
Figure 3 is a schematic longitudinal cross-section of a part of a device in accordance with the invention;
Figures 4a and 4b are schematic cross-sectional diagrams of a device in accordance with the invention under no compression and under compression respectively;
Figure 5 is a schematic diagram of a device in accordance with the invention in longitudinal cross-section;
Figure 6 is an exploded schematic diagram of a device in accordance with the invention;
Figure 7 is a longitudinal cross-section showing the liquid feed and return tubes and the air feed tube of a device in accordance with the invention;
Figure 8 is a schematic diagram showing the outer jacket of a device in accordance with the invention;
Figure 9 is a schematic diagram showing an alternative embodiment to that of Figure 8;
Figure 10 is a perspective view of an articulation coil for use in a device according to the invention,
Figure 11 shows an isometric image of an alternative device according to the invention, and
Figure 12 shows various cross-sections of the device of Figure 11.

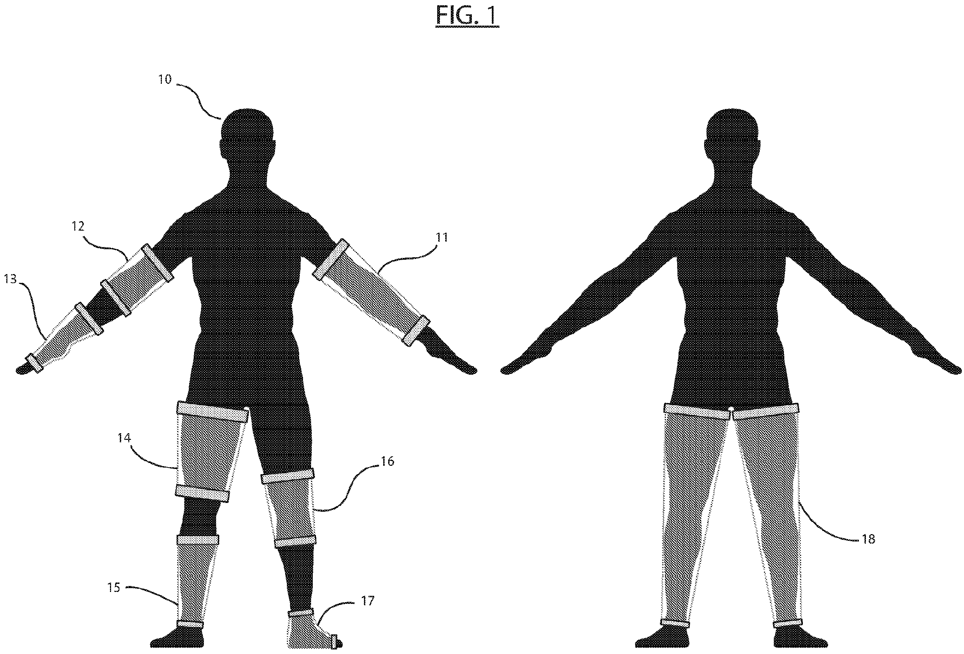

Figure 1 shows a patient 10 wearing various embodiments of a tubular cuff (which will be described in more detail with reference to later Figures). The various cuffs can be constructed so as to fit on the patient's arm 11, elbow 12, hand/wrist 13, upper leg 14, lower leg 15, knee 16, foot/ankle 17 or on both of the patient's legs 18 at the same time (the so-called "recovery trousers" embodiment). It will be appreciated that all of these embodiments are tubular in construction and therefore that the body part to be treated can be entirely surrounded by the tubular cuff by placing the body part inside the hollow part of the tube.

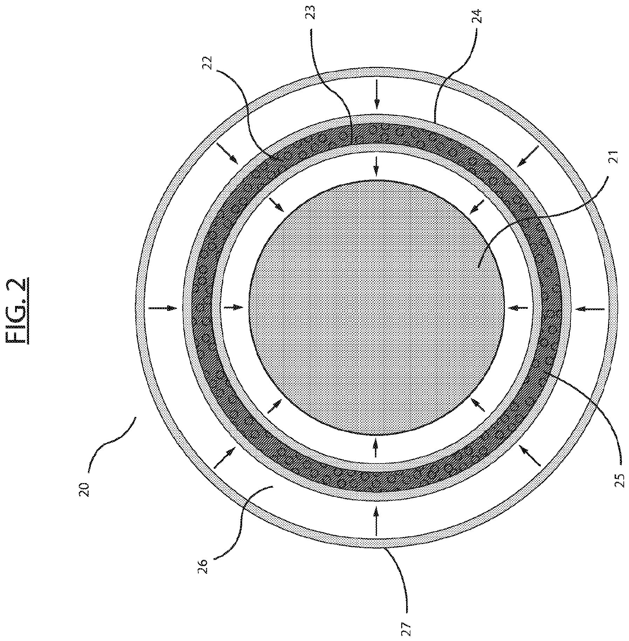

Turning to Figure 2, tubular cuff 20 comprises a thermal chamber 22 which is defined by the cylindrical space between cylindrical inner wall 23 and cylindrical separator wall 24. Pressure chamber 26 is defined by the space between separator wall 24 and outer wall 27, such that pressure chamber 26 completely surrounds thermal chamber 22 and is concentric with it. The hollow space defined by tubular inner wall 23 is the space into which body part 21 is placed prior to treatment.

It can be seen that thermal chamber 22 is filled with open-celled foam 25 (specifically, 6mm reticulated polyurethane foam with a porosity of 10 pores per inch) and the function of this will be described below.

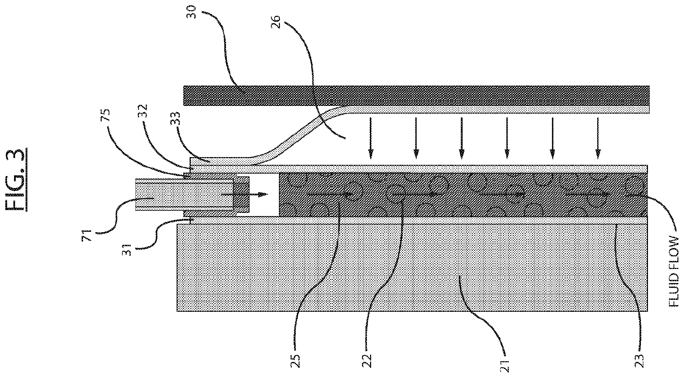

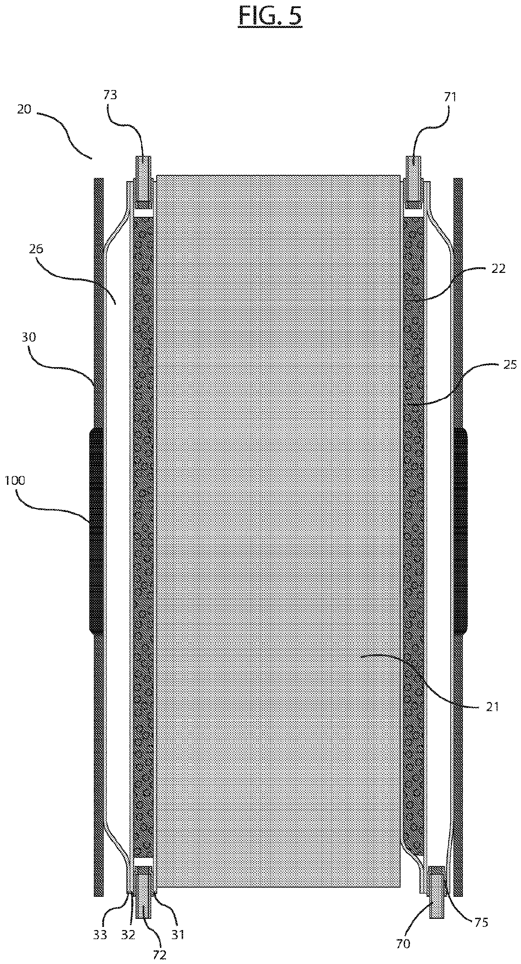

Turning now to Figures 5 and 3, these show a longitudinal cross-section of tubular cuff 20 (Figure 5) and an enlarged part of Figure 5 (Figure 3). It can be seen that inner wall 23, separator wall 24 and outer wall 27 are formed from three tubes 31, 32 and 33 respectively and that these are bonded together at either end of cuff 20 in order to terminate thermal chamber 22 and pressure chamber 26. The tubes may be hollow to enable fluid to be conveyed along them or alternatively may simply be formed of a flexible solid material to function solely as a wall. In the embodiment shown in Figures 3 and 5, inner wall 23 and separator wall 24 are formed from for 80-150 micron polyurethane film and outer wall 27 is formed from 200 denier polyurethane-coated nylon.

As shown in Figures 3, 5, 6 and 7, thermal chamber 22 and pressure chamber 26 are supplied with cooling/heating liquid and air respectively through the ends of tubular cuff 20. Air feed tube 70 is connected to pressure chamber 26 via tubing port 75 and likewise liquid feed tube 71, liquid return tube 72 and restricted liquid return tube 73 are connected to thermal chamber 22 via tubing ports 75. Tubing ports 75 are welded in between the layflat layers. This configuration eliminates the need for tubing within the liquid chamber, which can distort the cuff.

There are two liquid returns: liquid return tube 72 at the bottom of cuff 20 and restricted liquid return tube 73 at the top of cuff 20. Restricted liquid return tube 73 connects onto a smaller orifice size and functions as an exhaust to allow any air to escape from the thermal chamber 22. This has been found to prevent air from restricting liquid flow and preventing liquid from exiting cuff 20 freely. Restricted liquid return tube 73 also allows cuff 20 to continue its operation even if held upside down or in the opposite direction to what is recommended without putting too much strain on the hardware which provides the cooling/heating liquid.

In an alternative embodiment, tube 73 is a liquid feed tube rather than a restricted liquid return tube.

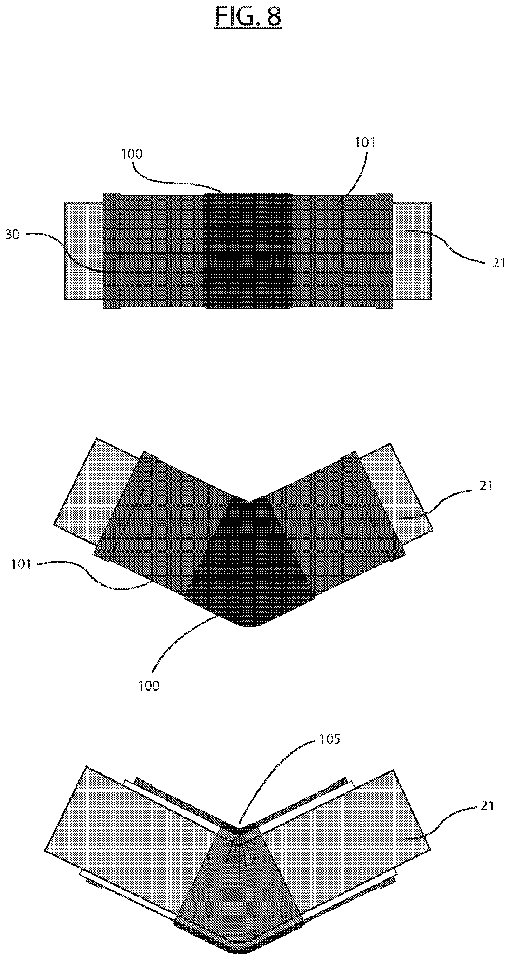

Cuff 20 may be fitted into outer jacket 30 having articulation area 100 and this is shown in more detail in Figure 8.

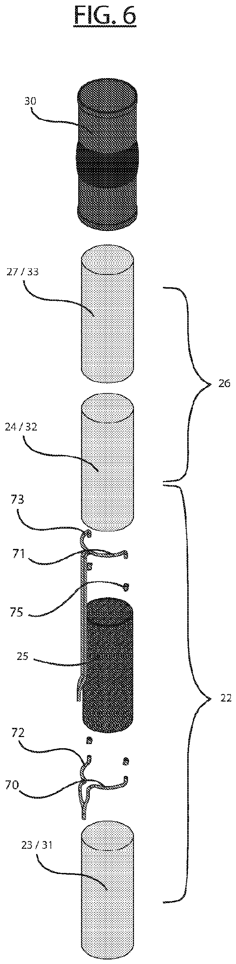

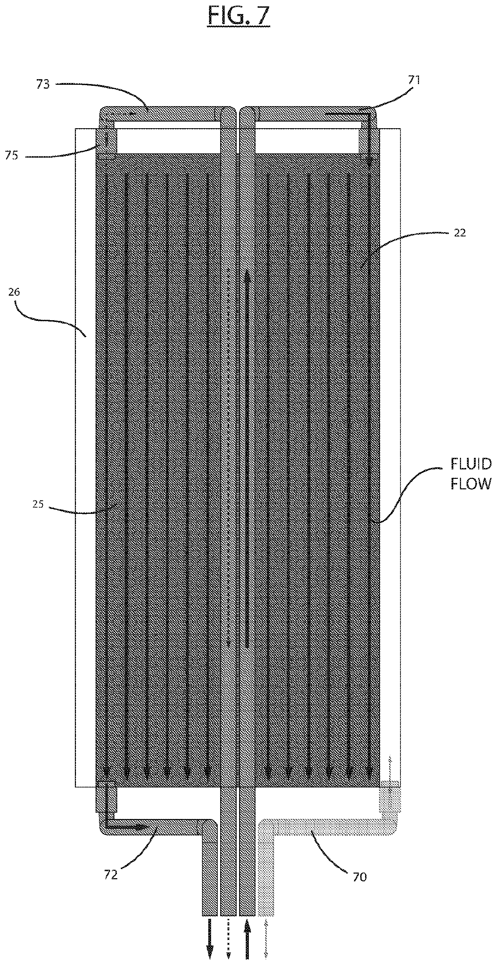

An alternative view of cuff 20 is shown in Figure 6 in which it can be seen that cuff 20 is formed from a series of concentric tubes fitted one inside the other, namely inner wall 23, open celled foam 25, separator wall 24, outer wall 27 and outer jacket 30 from bottom to top (or from inside to outside) in Figure 6. Figure 6 also shows air feed tube 70, liquid feed tube 71, liquid return tube 72, restricted liquid return tube 73 and tubing ports 75 which are shown in more detail in Figure 7.

In use, body part 21 is placed inside cuff 20 in the tubular space defined by inner wall 23. Pressure chamber 26 is then partially inflated by pumping air into it through air feed tube 70. Pressure chamber 26 is not fully inflated, but merely filled with sufficient air to enable cuff 20 to grip body part 21. This also has the effect of compressing thermal chamber 22 against body part 21 in order to maximise the surface contact between inner wall 23 and the skin of patient 10.

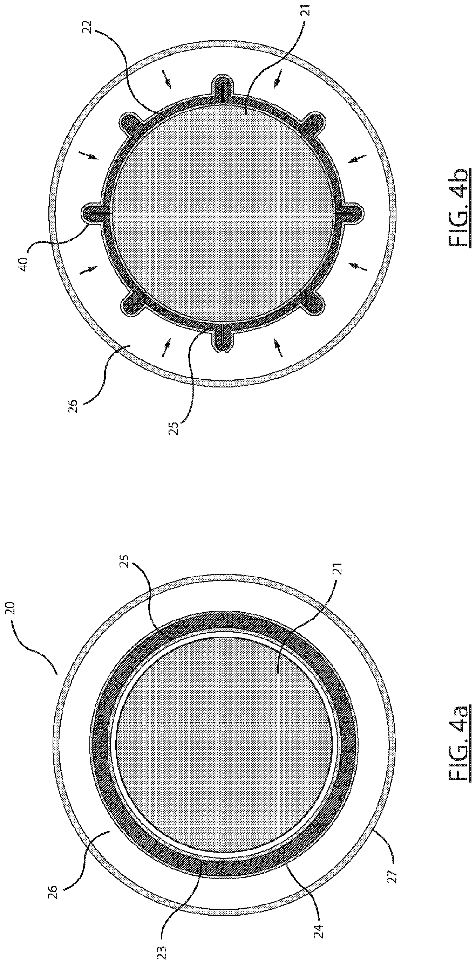

A key technical advantage of this construction is that any excess material formed of inner wall 23, open-celled foam 25 and separator wall 24 is automatically accommodated by pressure chamber 26 to enable thermal chamber 22 to surround and contact body part 21 completely without flow of liquid within thermal chamber 22 being impeded. This is shown schematically in Figures 4a and 4b, in which Figure 4a shows cuff 20 under no compression and Figure 4b shows cuff 20 under compression caused by pressurised air in pressure chamber 26 compressing thermal chamber 22 against body part 21. It can be seen that thermal chamber 22 completely contacts and surrounds body part 21 and that the parts of thermal chamber 22 which are in excess are accommodated within pressure chamber 26 in the form of ruckles 40. It is preferable for the material defining thermal chamber 22 to be sufficiently thin to allow it to collapse into itself so that the excess material can move into the space of the pressure chamber 26 to form ruckle 40.

Once thermal chamber 22 has been contoured around body part 21, it is filled with cooling fluid as will now be described.

Thermal chamber 22 is filled with cooling fluid by injecting the fluid into liquid feed tube 71. It then flows through open-celled foam 25 from one end of thermal chamber 22 to the other and then exits thermal chamber 22 via liquid return tube 72.

The function of open-celled foam 25 is randomly and evenly to distribute the fluid throughout the thermal chamber 22 so that the cooling effect is applied evenly and completely over the surface area of body part 21 which is contacted by thermal chamber 22. Foam 25 also takes up a large volume inside thermal chamber 22, which means that not so much cooling fluid is required to 'submerse' the limb in liquid. It also maintains the shape and structure of the thermal chamber 22, ensuring that fluid flow is not restricted to particular areas. Foam 25 can compress, deform and stretch to conform evenly around the body part. Different densities, thicknesses and weights of foam 25 can be used to improve coolant flow and thermal transfer.

Once thermal chamber 22 has been filled with cooling fluid, further pressure can be applied to the air in pressure chamber 26 in order to maximise compression of thermal chamber 22 against body part 21. This can also be used to apply compression therapy to body part 21, and this compression therapy can be varied by varying the pressure of air in pressure chamber 26.

It will be appreciated that a heating fluid can be used instead of a cooling fluid for applying heat therapy to body part 21.

When the therapy has been completed, flow of the cooling/heating fluid through thermal chamber 22 is ceased and air pressure is released by emptying the pressure chamber 26 so that cuff 20 can be removed from body part 21.

As noted below, cuff 20 is fitted inside outer jacket 30, preferably prior to being fitted onto body part 21. Alternatively, outer jacket 30 can be applied over cuff 20 once it has been placed over body part 21.

Outer jacket 30 is formed of any suitable textile such as ripstop nylon or coated fabric. The function of outer jacket 30 is to provide cylindrical support and to direct all compression forces inwardly onto body part 21. It also restricts and controls the amount of outward ballooning from pressure chamber 26.

As shown in Figure 8, outer jacket 30 can include an elastic section 100 and a more rigid material 101 in order to provide an articulation area 105 around an articulated body part 21. This allows for articulation of the body part and allows the device to bend laterally. It also allows the pressure chamber 26 to balloon and deform around the articulated joint whereas this ballooning and deforming is restricted by rigid material 101 around the straight/non-articulated parts of the limb.

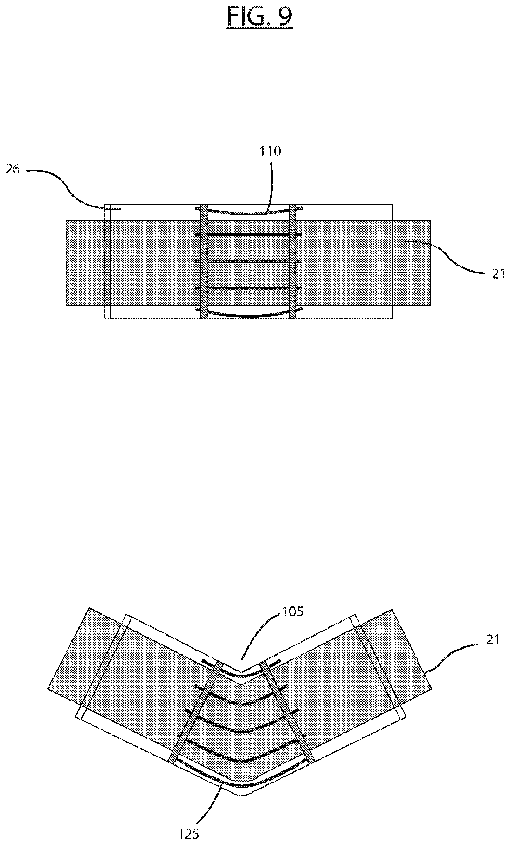

Figure 9 shows an alternative embodiment which provides for an articulation area 105 as in the embodiment of Figure 8. In this embodiment, the pressure chamber 26 is provided with plastic ligaments 110 which can stretch in a region 115 on the outside of the articulated body part to control deformation of the device as a whole. Ligaments 110 may alternatively be provided as a part of outer jacket 30 (not shown in Figure 9).

It will be understood that different outer jackets 30 can be provided for different body parts 21, depending upon the size, shape and articulation required and that these specific outer jackets 30 can be used with generic cuffs 20.

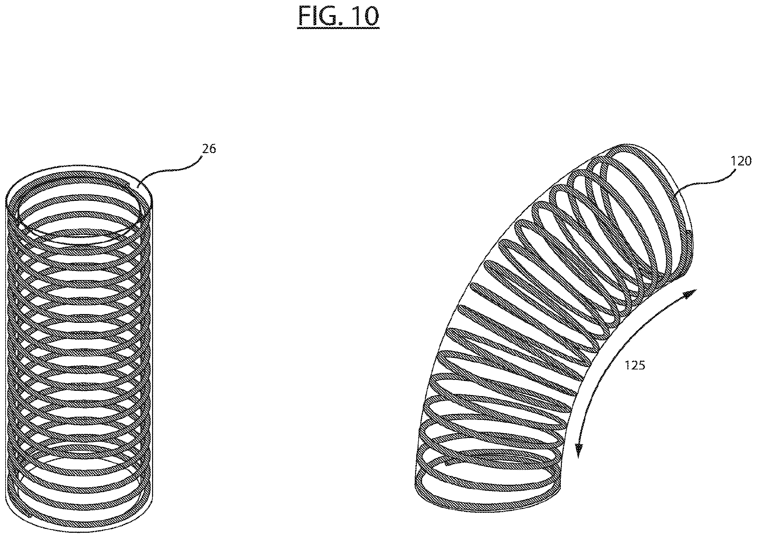

Optionally, cuff 20 may include articulation coil 120 as shown in Figure 10. This is formed of a helical coil of reinforcement wire and is housed in pressure chamber 26. Its function is to provide radial support whilst allowing lateral flex 125 and thereby accommodate limb flexion.

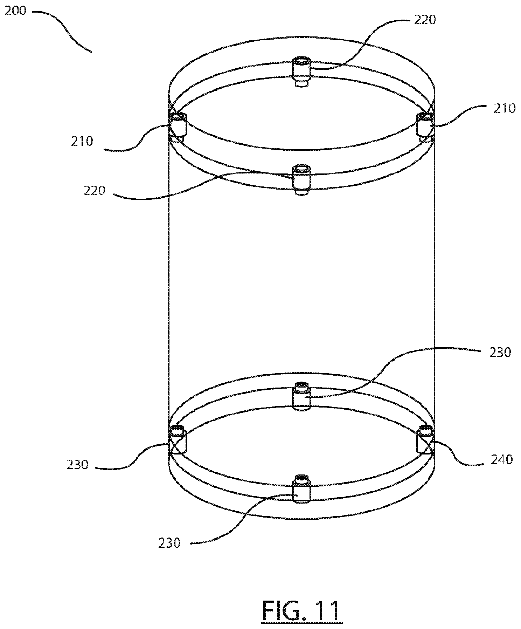

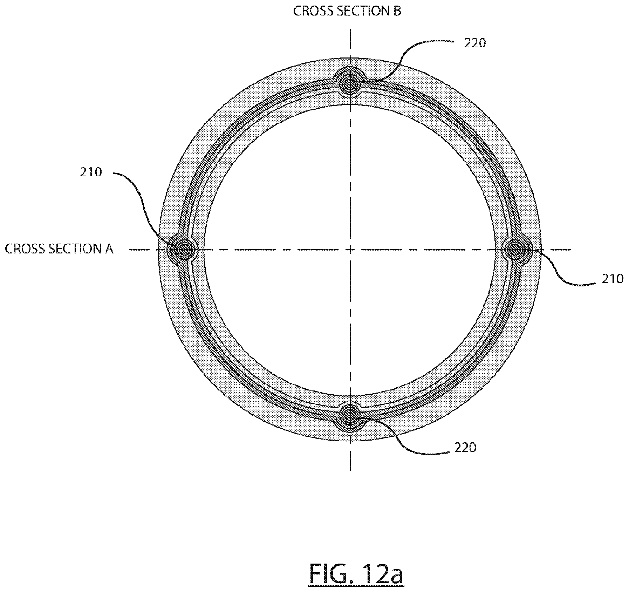

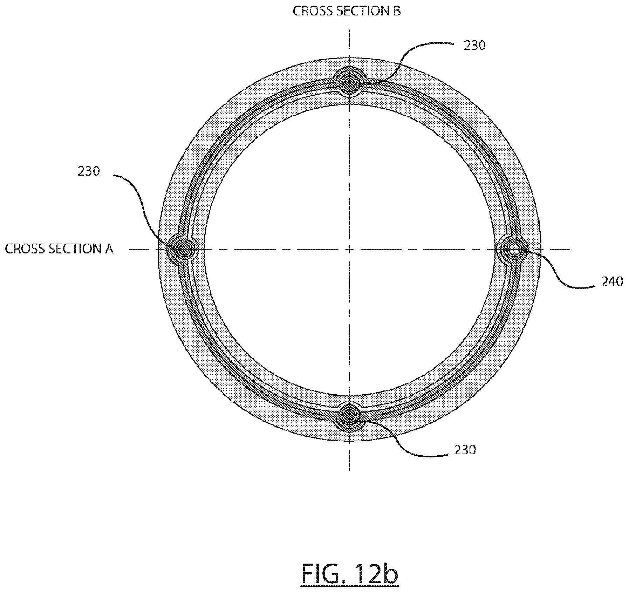

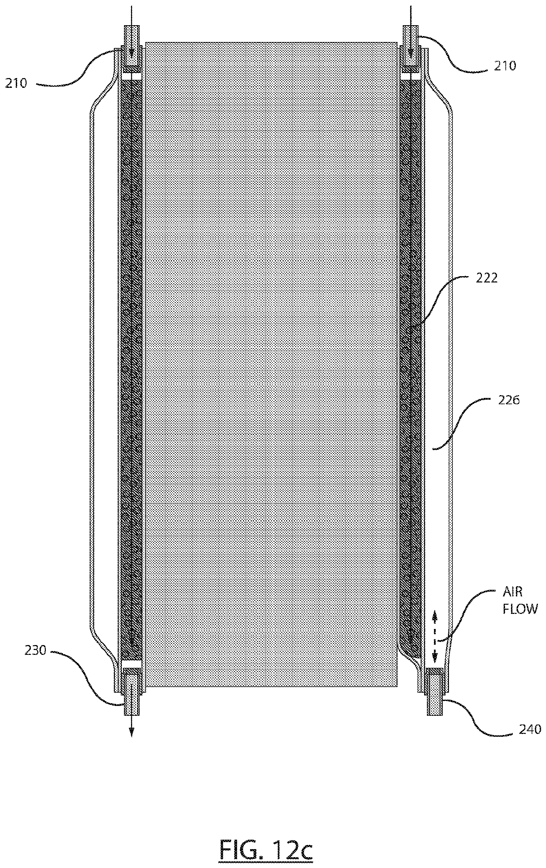

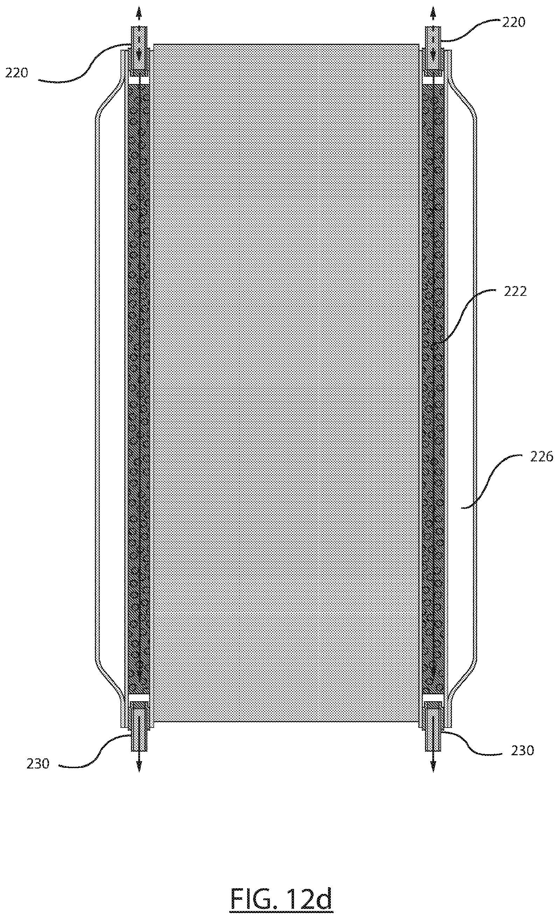

An alternative tubular cuff 200 is shown in Figures 11 and 12. Cuff 200 has 8 ports in total: 2 liquid feed and 2 restricted liquid return ports at the top, and 3 liquid return and 1 air feed at the bottom. Cuff 200 has thermal chamber 222 which is constructed as described for thermal chamber 22 above and pressure chamber 226 which is constructed as described for pressure chamber 26 above.

In more detail, Fig. 11 shows an isometric view of cuff 200 having two liquid feed ports 210 and two restricted return ports 220 at the top of cuff 200, and three liquid return ports 230 and one air feed port 240 at the bottom of cuff 200.

Cuff 200 is illustrated in more detail in top cross section in Fig. 12A and in bottom cross section in Fig. 12B. Fig. 12C then shows cross section view A and Fig. 12D shows cross section view B through Figures 12A and 12B.

In use, heating or cooling liquid is supplied to the cuff via liquid feed ports 210 and flows through the thermal chamber 222 as before, to exit via liquid return ports 230. Restricted return ports 220 function as an exhaust to allow any air to escape from the thermal chamber 222 as above. Air is supplied to pressure chamber 226 as described above.

In a preferred embodiment, any port configuration could be used as long as there is a minimum of 1 liquid feed at the top with 1 liquid return and 1 air at the bottom. The restricted return ports at the top could be replaced with addition liquid feeds, or there could be a combination of both liquid feeds and returns at both ends.

## Claims

1. A device for heating or cooling a body part, comprising
a body (20) having an elongate treatment cavity (21) into which a body part can be placed for treatment,
a first chamber (22) having an inlet (71) and an outlet (72) for a heating or cooling fluid, wherein the first chamber is formed by a space between an inner wall (23) of the first chamber in the form of a cylindrical surface and an outer wall (24) of the first chamber in the form of a cylindrical surface,
wherein the inner wall of the first chamber forms the external boundary of at least part of the treatment cavity, whereby heating or cooling fluid in the first chamber heats or cools a body part in the treatment cavity,
and wherein the treatment cavity is open at both ends so that in use a body part can be placed into one end of the treatment cavity and emerge from the other end, wherein the device additionally comprises means for compressing, in use, the first chamber into the body part, wherein the means for compressing comprises a second chamber (26) completely surrounding the first chamber and having an inlet (71) and an outlet (70) for a pressurising fluid, wherein the second chamber is formed by a space between an inner wall (24) of the second chamber in the form of a cylindrical surface and an outer wall (27) of the second chamber in the form of a cylindrical surface, whereby putting said fluid under pressure in the second chamber compresses the first chamber into the body part.

2. A device as claimed in claim 1, wherein the inner wall and the outer wall of the first chamber are concentric.

3. A device as claimed in any preceding claim, wherein the inner wall of the first chamber is formed from an integral cylindrical surface.

4. A device as claimed in any preceding claim, wherein the outer wall of the first chamber is formed from an integral cylindrical surface.

5. A device as claimed in any preceding claim, wherein the first chamber continuously surrounds the treatment cavity.

6. A device as claimed in any preceding claim, wherein the inner wall and the outer wall of the second chamber are concentric.

7. A device as claimed in any preceding claim, wherein the inner wall of the second chamber is formed from the outer wall of the first chamber.

8. A device as claimed in any preceding claim, wherein said means for compressing comprises a thicker or relatively inelastic outer wall for the first chamber, which forces the first chamber into the body part when the first chamber is pressurised.

9. A device as claimed in any preceding claim, wherein the first chamber includes a material (25) through which the heating or cooling fluid can flow in use.

10. A device as claimed in claim 9, wherein the material has multiple pathways therethrough for the first fluid which enable even distribution of the said fluid through the chamber.

11. A device as claimed in claim 10, wherein said pathways are equally deformable.

12. A device as claimed in any of claims 9 to 11, wherein said material comprises an open-celled foam, a 3D spacer material, a plurality of particles, or a fluid.

## Patentansprüche

1. Vorrichtung zum Erwärmen oder Abkühlen eines Körperteils, aufweisend
einen Körper (20), der einen länglichen Behandlungshohlraum (21) aufweist, in den ein Körperteil zur Behandlung plaziert werden kann,
eine erste Kammer (22), die einen Einlass (71) und einen Auslass (72) für ein wärmendes oder kühlendes Fluid aufweist, wobei die erste Kammer gebildet wird von einem Raum zwischen einer inneren Wandung (23) der ersten Kammer in der Form einer zylindrischen Oberfläche und einer äußeren Wandung (24) der ersten Kammer in der Form einer zylindrischen Oberfläche
wobei die innere Wandung der ersten Kammer eine äußere Grenze von zumindest einem Teil des Behandlungshohlraums bildet, wodurch wärmendes oder kühlendes Fluid in der ersten Kammer einen Körperteil in dem Behandlungshohlraum wärmt oder kühlt,
und wobei der Behandlungshohlraum an beiden Enden offen ist, so dass bei Benutzung ein Körperteil in ein Ende des Behandlungshohlraums platziert werden kann und aus dem anderen Ende hervorragen kann,
wobei die Vorrichtung zusätzlich Mittel zum Komprimieren, während der Benutzung, der ersten Kammer in den Körperteil aufweist, wobei die Mittel zum Komprimieren eine zweite Kammer (26) aufweisen, welche die erste Kammer vollständig umgibt und einen Einlass (71) und einen Auslass (70) für ein Druck übertragendes Fluid aufweist, wobei die zweite Kammer gebildet wird von einem Raum zwischen einer inneren Wandung (24) der zweiten Kammer in der Form einer zylindrischen Oberfläche und einer äußeren Wandung (27) der zweiten Kammer in der Form einer zylindrischen Oberfläche, wobei unter Druck Setzen der Flüssigkeit in der zweiten Kammer die erste Kammer in den Körperteil komprimiert.

2. Vorrichtung gemäß Anspruch 1, wobei die innere Wandung und die äußere Wandung der ersten Kammer konzentrisch sind.

3. Vorrichtung gemäß einem der vorangehenden Ansprüche, wobei die innere Wandung der ersten Kammer aus einer integralen zylindrischen Oberfläche gebildet ist.

4. Vorrichtung gemäß einem der vorangehenden Ansprüche, wobei die äußere Wandung der ersten Kammer aus einer integralen zylindrischen Oberfläche gebildet ist.

5. Vorrichtung gemäß einem der vorangehenden Ansprüche, wobei die erste Kammer den Behandlungshohlraum zusammenhängend umgibt.

6. Vorrichtung gemäß einem der vorangehenden Ansprüche, wobei die innere Wandung und die äußere Wandung der zweiten Kammer konzentrisch sind.

7. Vorrichtung gemäß einem der vorangehenden Ansprüche, wobei die innere Wandung der zweiten Kammer aus der äußeren Wandung der ersten Kammer gebildet ist.

8. Vorrichtung gemäß einem der vorangehenden Ansprüche, wobei die Mittel zum Komprimieren eine dickere oder relativ inelastische äußere Wandung der ersten Kammer aufweisen, welche die äußere Kammer in den Körperteil drückt, wenn die erste Kammer unter Druck gesetzt wird.

9. Vorrichtung gemäß einem der vorangehenden Ansprüche, wobei die erste Kammer ein Material (25) aufweist, durch welches das wärmende oder kühlende Fluid in Benutzung strömen kann.

10. Vorrichtung gemäß Anspruch 9, wobei das Material mehrere durch es hindurch gehende Pfade für das erste Fluid aufweist, welche eine gleichmäßige Verteilung des besagten Fluids durch die Kammer ermöglichen.

11. Vorrichtung gemäß Anspruch 10, wobei Pfade gleichermaßen deformierbar sind.

12. Vorrichtung gemäß einem der Ansprüche 9 bis 11, wobei das Material einen offenzelligen Schaum, ein 3D Abstandhaltermaterial, eine Vielzahl an Partikeln oder ein Fluid umfasst.

## Revendications

1. Dispositif pour chauffer ou refroidir une partie de corps, comprenant
un corps (20) ayant une cavité de traitement allongée (21) dans laquelle une partie de corps peut être placée pour traitement,
une première chambre (22) ayant un orifice d'entrée (71) et un orifice de sortie (72) pour un fluide de chauffage ou de refroidissement, dans lequel la première chambre est formée par un espace entre une paroi interne (23) de la première chambre sous la forme d'une surface cylindrique et une paroi externe (24) de la première chambre sous la forme d'une surface cylindrique,
dans lequel la paroi interne de la première chambre forme la limite externe d'au moins une partie de la cavité de traitement de telle sorte qu'un fluide de chauffage ou de refroidissement dans la première chambre chauffe ou refroidisse une partie de corps dans la cavité de traitement,
et dans lequel la cavité de traitement est ouverte au niveau des deux extrémités de telle sorte que, lors de l'utilisation, une partie de corps puisse être placée dans une extrémité de la cavité de traitement et sortir de l'autre extrémité,
dans lequel le dispositif comprend en plus un moyen pour comprimer, lors de l'utilisation, la première chambre dans la partie de corps, dans lequel le moyen de compression comprend une seconde chambre (26) entourant complètement la première chambre et ayant un orifice d'entrée (71) et un orifice de sortie (70) pour un fluide de mise sous pression, dans lequel la seconde chambre est formée par un espace entre une paroi interne (24) de la seconde chambre sous la forme d'une surface cylindrique et une paroi externe (27) de la seconde chambre sous la forme d'une surface cylindrique, de telle sorte que la mise sous pression du fluide dans la seconde chambre comprime la première chambre dans la partie de corps.

2. Dispositif selon la revendication 1, dans lequel la paroi interne et la paroi externe de la première chambre sont concentriques.

3. Dispositif selon l'une quelconque des revendications précédentes, dans lequel la paroi interne de la première chambre est formée à partir d'une surface cylindrique intégrale.

4. Dispositif selon l'une quelconque des revendications précédentes, dans lequel la paroi externe de la première chambre est formée à partir d'une surface cylindrique intégrale.

5. Dispositif selon l'une quelconque des revendications précédentes, dans lequel la première chambre entoure de façon continue la cavité de traitement.

6. Dispositif selon l'une quelconque des revendications précédentes, dans lequel la paroi interne et la paroi externe de la seconde chambre sont concentriques.

7. Dispositif selon l'une quelconque des revendications précédentes, dans lequel la paroi interne de la seconde chambre est formée à partir de la paroi externe de la première chambre.

8. Dispositif selon l'une quelconque des revendications précédentes, dans lequel ledit moyen de compression comprend une paroi externe plus épaisse ou relativement rigide pour la première chambre qui force la première chambre dans la partie de corps lorsque la première chambre est mise sous pression.

9. Dispositif selon l'une quelconque des revendications précédentes, dans lequel la première chambre comprend un matériau (25) à travers lequel le fluide de chauffage ou de refroidissement peut s'écouler lors de l'utilisation.

10. Dispositif selon la revendication 9, dans lequel le matériau comporte de multiples passages à travers ce dernier pour le premier fluide qui permettent une distribution égale dudit fluide à travers la chambre.

11. Dispositif selon la revendication 10, dans lequel lesdits passages peuvent être déformés de manière égale.

12. Dispositif selon l'une quelconque des revendications 9 à 11, dans lequel ledit matériau comprend une mousse à cellules ouvertes, un matériau d'espacement 3D, une pluralité de particules ou un fluide.
